# EUROPÄISCHE PATENTSCHRIFT

(11) **EP 1 175 181 B1**
(45) Veröffentlichungstag und Bekanntmachung des Hinweises auf die Patenterteilung: **12.01.2005**
(21) Anmeldenummer: 00918656.0
(22) Anmeldetag: 03.05.2000
(51) Int. Cl.: A61B 17/80

(54) **VERBLOCKBARE KNOCHENPLATTE**
BLOCKABLE BONE PLATE
PLAQUE POUR OSTEOSYNTHESE POUVANT ETRE BLOQUEE

(30) Priorität: 03.05.1999 CH 83099
(43) Veröffentlichungstag der Anmeldung: 30.01.2002
(73) Patentinhaber: Medartis AG, 4051 Basel (CH)
(72) Erfinder: PFEFFERLE, Joachim, D-79244 Münstertal (DE); ROTH, Michael, D-79110 Freiburg (DE); SCHEUBLE, Peter, D-79241 Wasenweiler (DE); ZEUNER, Hermann, D-79106 Freiburg (DE)
(74) Vertreter: Heinen, Detlef
(86) Internationale Anmeldenummer: PCT/CH2000/000247
(87) Internationale Veröffentlichungsnummer: WO 2000/066012

(56) Entgegenhaltungen:
- DE-A- 19 629 011
- DE-A- 19 858 889
- US-A- 5 709 686
- US-A- 5 807 396

## Beschreibung

### Anwendungsgebiet der Erfindung

Die vorliegende Erfindung bezieht sich auf eine verblockbare Knochenplatte mit gesicherten Knochenschrauben, die insbesondere als Rekonstruktionssystem für den maxillofacialen Bereich des menschlichen Schädels, z.B. für den Unterkiefer, dienen. Bis auf weiteres werden verkürzt die Begriffe "Platte", "Schraube" und "Platten-Schrauben-Verbindung" verwendet. Derartige Platten setzt man beispielsweise ein, um geschwächte, zurückgebildete Knochenstrukturen zu verstärken. Bei grossen Kontinuitätsdefekten müssen die Platten über längere Zeiträume Stabilität bewirken und die Belastungen aufnehmen, welche der fehlende Knochen zu tragen hätte. Platten dieser Art bestehen in der einfachsten Form aus einem geraden, langgestreckten Ast mit einer Vielzahl von Plattengliedern, in denen jeweils ein Schraubenloch zur Aufnahme einer Schraube vorhanden ist. Die Platte lässt sich nach der jeweils erforderlichen Länge zuschneiden, was durch Abtrennen überzähliger Plattenglieder geschieht, und zur Anpassung an die örtliche anatomische Situation biegen.

An die Knochenplatte bzw. eine Platten-Schrauben-Verbindung werden folgende Anforderungen gestellt:
- hohe Festigkeit und Stabilität der Knochenplatte;
- hohe Steifigkeit der Platten-Schrauben-Verbindung;
- Biegsamkeit der Platte in allen Ebenen bei geringem Festigkeitsverlust sowohl in der Plattenebene als auch um die Plattenebene, einschliesslich als Torsion;
- Möglichkeit des Anlegens der verschiedenen biologischen Gewebearten an der Platte;
- möglichst geringe Flächenpressung der Platte auf der Knochenhaut; und
- Sicherung (sogenannte Verblockung) der die Platte durchdringenden Schrauben, um ein selbständiges Lösen der Schrauben zu unterbinden.

### Stand der Technik

Um die Flächenpressung der Platte auf die Knochenhaut gering zu halten, schlägt die US 5,810,823 vor, an der Plattenunterseite einsetzbare oder feststehende Distanzelemente mit einem Innengewinde anzuordnen, die auf dem Knochen aufsitzen und von den Gewindeschäften der Schrauben durchdrungen werden. Variabel positionierbare Distanzelemente erlauben auch eine von der Senkrechten abweichende Ausrichtung der Schrauben. Zwar wird mit dieser Konstruktion das grossflächige Aufpressen der Platte auf die Knochenhaut vermieden, erhöhter Druck entsteht jedoch an den Aufsetzstellen der Distanzelemente. Die relativ kleinen einsetzbaren Distanzelemente verkomplizieren das chirurgische Prozedere. Unabhängig davon, ob die Distanzelemente fest oder einsetzbar sind, bewirken sie keine verbesserte Sicherung gegen unbeabsichtigtes Lösen der Schrauben.

In der WO 96/39975 wird eine verblockte Platten-Schrauben-Verbindung beschrieben, wo an der Plattenunterseite, in den Bereich der Schraubenlöcher einsetzbare, versenkte Sicherungselemente vorgesehen sind. Durch das einzelne Sicherungselement ragt die gewindelose Halspartie des Schraubenschafts, welcher einen geringeren Durchmesser als das Gewindeteil aufweist. Zunächst lässt sich das Gewindeteil durch das Sicherungselement hindurchstecken und in den Knochen einschrauben, wobei der Schraubenkopf in einer Ansenkung an der Plattenoberseite zu liegen kommt. Nach einer Wärmebehandlung schrumpft das Sicherungselement, so dass die Platte beabstandet zur Knochenhaut gehalten wird und die Schraube gegen Herausdrehen gesichert ist. Dieses System benötigt die speziellen Sicherungselemente sowie Geräte und ist daher im Handling und in der Herstellung aufwendig.

In der Firmenschrift "SYNTHES® - THORP-Rekonstruktionsset" der STRATEC Medical, Waldenburg/CH, ist ein Plattensystem offenbart, wo durch die Schraubenlöcher der Platte zunächst Verankerungsschrauben in den Knochen eingeschraubt werden. In den geschlitzten Kopf der Verankerungsschraube, der im Schraubenloch sitzt, dreht man eine Spreizschraube ein. Hierdurch presst sich der Kopf der Verankerungsschraube gegen die Wandung des Schraubenlochs in der Platte. Somit ist die Verankerungsschraube gegen Herausdrehen gesichert und die Platte wird quasi beabstandet zur Knochenhaut gehalten. Damit sich die Schraubenlöcher in der Platte beim Biegen nicht verengen, sind Biegeeinsätze als einsteckbare Kerne vorgesehen. Die höhere Anzahl von Teilen erfordert während der Operation einen vermehrten Zeit- und instrumentellen Aufwand und gestaltet sich insgesamt diffiziler.

Eine Weiterentwicklung einer verblockbaren Platten-Schrauben-Verbindung ist aus der US 5,709,686 bekannt. Die Knochenplatte besitzt mehrere in Richtung der Plattenlängsachse vorgesehene Löcher zum Durchtritt von Schrauben. Das Schraubenloch ist oval ausgebildet, wobei dessen Hauptachse in der Plattenlängsachse liegt. An der Plattenoberseite ist das Schraubenloch von einer sphärischen Ansenkung umgeben. Über die kürzere Nebenachse des Schraubenlochs ist ein partielles Innengewinde vorhanden, dessen Gewindegänge jeweils zur Hauptachse des ovalen Schraubenlochs hin auslaufen. Das Innengewinde dient der Aufnahme einer an der Schraube unter deren Kopf liegender Gewindepartie, welche im Durchmesser gegenüber dem Gewindeschaft der Schraube erweitert ist. Das Schraubenloch öffnet sich zur Plattenunterseite hin im Bereich der beiden Enden der Hauptachse, d.h. ausserhalb des Innengewindes auf der Plattenlängsachse.

Bei der klinischen Applikation der Platte ragt der Gewindeschaft der Schraube in den Knochen hinein, während die Gewindepartie mit dem Innengewinde im Schraubenloch in Eingriff kommt. Die Platte wird dadurch abgestützt und nicht von der Zugkraft der Schraube auf die Knochenhaut gepresst. Gegen unbeabsichtigtes Herausdrehen ist die Schraube durch die Gewindeverbindung zwischen dem Innengewinde im Schraubenloch und der Gewindepartie der Schraube gesichert. Platte und Schraube sind verblockt. Die Ansenkung um das Schraubenloch erlaubt bei exzentrischem Ansatz einer Schraube ohne Gewindepartie, eine Kompression zwischen Knochenkompartimenten zu erzeugen. Schrauben ohne eine Gewindepartie zur Verblockung lassen sich auch abweichend von der Senkrechten eindrehen. Grösserer Freiraum hierfür besteht in Richtung der Plattenlängsachse durch die ovale Lochform und das nur partielle Innengewinde im Schraubenloch.

Mit der Platten-Schrauben-Verbindung gemäss der US 5,709,686 ist ein einwandfreies Verblocken und eine zusätzliches Sicherung der Schrauben möglich, es verbleiben aber markante Nachteile, wie:
- Zur Verhinderung von Deformationen des Innengewindes in den Schraubenlöchern beim Biegen der Platte müssen weiterhin Biegeeinsätze verwendet werden.
- Bereits bei geringen Drehmomenten während des Eindrehens der Schraube kann das Innengewinde im Schraubenloch überdreht werden. Mit Standard-Schraubendrehern können solche kritischen Drehmomente ohne weiteres aufgebracht werden. Eine beschädigte Platte ist unbrauchbar; man muss eine neue Platte und eventuell auch eine neue Schraube verwenden.
- Bei einer verblockten Platten-Schrauben-Verbindung können die eingedrehten Schrauben nur senkrecht positioniert werden, was den Einsatz von exakt senkrecht stehenden Bohrerführungen erforderlich macht.
- Aufgrund der gesamten Geometrie der Plattenlöcher muss die Bearbeitung von zwei Richtungen erfolgen, was die Plattenherstellung aufwendiger macht.

### Aufgabe der Erfindung

Angesichts der aufgezeigten Unvollkommenheiten der bis dato bekannten verblockbaren Knochenplatten mit gesicherten Schrauben, liegt der Erfindung die Aufgabe zugrunde, eine verbesserte verblockbare Platten-Schrauben-Verbindung vorzuschlagen. Hierbei kommt es insbesondere darauf an, ohne dritte Teile, wie Einsätze oder Spreizschrauben zur Verblockung und Biegeeinsätze, auszukommen. Die Verbindungen sollen gegen Überdrehen und Winkelversatz der Schrauben in Relation zur Senkrechten und der Plattenlängsachse weniger empfindlich sein. Wünschenswert ist, innerhalb der Platten-Schrauben-Verbindung für die Schrauben ein hohes Ausdrehmoment zu erreichen, um somit ein selbsttätiges Lösen der Schrauben strikter zu vermeiden. Die klinische Anwendung für den Operateur soll sich möglichst einfach gestalten. Erhalten bleiben müssen die Möglichkeiten, eine osteosynthetische Knochenkompression herbeizuführen sowie die Verbindung mit herkömmlichen Schrauben unverblockt anzuwenden. Schliesslich müssen sich die Teile der Verbindung auf effiziente Weise in Serie fertigen lassen.

### Übersicht über die Erfindung

Die erfindungsgemässe Weiterentwicklung der verblockbaren Knochenplatte basiert auf einer Knochenplatte und Schrauben mit einem Verblockungsgewinde gemäss der Gattung aus der US 5,709,686. Die Platte besteht aus mehreren Plattengliedern, welche durch Stege miteinander verbunden sind. Zumindest in einigen Plattengliedern, vorzugsweise in jedem Plattenglied, ist ein Schraubenloch vorhanden, das auf der Plattenoberseite von einer kugelförmigen Ansenkung umgeben wird. Intern ist im Schraubenloch eine Eingriffskontur vorhanden, die aus partiell an der Wandung des Schraubenlochs horizontal und radial umlaufenden Konturtälern und dazu benachbarten verbleibenden Konturspitzen besteht. Der Durchmesser der Ansenkung zur Aufnahme des Schraubenkopfes ist grösser als die lichte Weite der Eingriffskontur. Zu den Rändern der Eingriffskontur hin läuft diese aus, so dass dort glatte, unkonturierte Wandungsbereiche im Schraubenloch verbleiben. Im Verhältnis zum Gewindeschaft der Schraube stellt sich das Schraubenloch als Langloch dar. Die Eingriffskontur wird vorzugsweise durch Fräsen hergestellt und hat z.B. spitze, runde, trapezförmige oder sägezahnförmige Gestalt.

An der zur Verblockung geeigneten Schraube ist unterhalb des Schraubenkopfes ein Verblockungsgewinde vorhanden, das einen gleich grossen oder grösseren Durchmesser als das zum Eingriff in den Knochen bestimmte Gewinde am Gewindeschaft aufweist. Beim Fixieren der Platte am Knochen durchragt die Schraube mit ihrem Gewindeschaft das Schraubenloch und das Gewinde des Gewindeschafts dreht sich in den Knochen hinein. In der Schlussphase des Eindrehens der Schraube gelangt das Verblockungsgewinde unterhalb des Schraubenkopfes in die Eingriffskontur im Schraubenloch. Da das Verblockungsgewinde mit seinem wendelförmigen Verlauf und seiner Steigung in der Form zur Eingriffskontur nicht komplementär ist, kommt es zur Deformation an beiden und damit zu einer gegen Lösen widerstandsfähigeren Verbindung. Eine Verblockung von reduzierter Stärke lässt sich noch erzielen, wenn an der Schraube ein durchgängiges Gewinde vorhanden ist, dessen oberste Gewindegänge die Eingriffskontur verklemmt unterlaufen.

Besondere Vorteile bei der Osteosynthese von Frakturen an im Bogen verlaufender Knochen, z.B. ein Unterkiefer, ergeben sich bei Verwendung der erfindungsgemässen Verblockung an einer Knochenplatte mit kreisbogenförmiger Plattenlängsachse. Die Knochenplatte besteht zumindest aus einem kreisbogenförmigen Hauptsegment. Für spezielle Aufgaben ist das Hauptsegment mit einem ein- oder beidseitig daran ansetzenden, in der Plattenebene liegenden Lateralsegment versehen, welches sich gerade oder gebogen erstreckt. An der Knochenplatte können manche Schraubenlöcher nicht für die Verblockung, sondern als zylindrische Standard-Schraubenlöcher oder als richtungsorientierte Kompressionslöcher ausgestaltet sein. Vorzugsweise erhält die kreisbogenförmige Knochenplatte ihre Gestalt ohne Umformen, so dass die Platten ohne eine erste Schwächung durch Biegespannungen zum Chirurgen gelangen.

Dank der Erfindung steht jetzt eine verblockbare Platten-Schrauben-Verbindung mit folgenden Vorteilen zur Verfügung:
- In der klinischen Anwendung ist die Platten-Schrauben-Verbindung effizient, da keine dritten Teile zum Verblocken nötig sind. Auch beim Verbiegen der Platte werden keine Biegeeinsätze benötigt. Die Platte neigt dazu, sich im Bereich der die einzelnen Plattenglieder verbindenden Stege zu verbiegen. Aber selbst bei einer Biegung innerhalb des Schraubenlochs wird die Funktionsfähigkeit der Verblockung nicht beeinträchtigt.
- Bei der Verblockung wirken keine Kräfte auf die Knochenhaut ein, so dass diese geschont wird. Die Platte lässt sich ähnlich einem Fixateur, beabstandet zum Knochen positionieren.
- Die interne Eingriffskontur im Schraubenloch erlaubt auch einen leichten Winkelversatz der eingedrehten Schraube in Relation zur Senkrechten und Plattenlängsachse. Dies ohne Funktionsverlust der Verblockung.
- Bei Erzeugung einer Kompression werden die Schrauben nicht auf Zug beansprucht, da durch die Verblockung der Schraube in der Platte keine Axialbewegung zwischen Platte und Knochen zugelassen wird.
- Gegen unbeabsichtigtes Lösen sind die eingedrehten Schrauben besser gesichert, da ein hohes Ausdrehmoment aufgebracht werden muss. Dies infolge der Deformationen bei der Verblockung an der internen Eingriffskontur am Schraubenloch und am beteiligten Gewinde der Schraube.
- Die Gefahr des Überdrehens und damit einer Beschädigung der Platte ist verringert. Bei herkömmlichen Platten überdrehen die Schrauben bereits bei geringeren Eindrehmomenten, die mit Standardschraubendrehern mühelos aufgebracht werden können.
- Die in den Schraubenlöchern intern angeordnete Eingriffskontur verursacht eine wesentlich geringere Kerbwirkung als herkömmliches Innengewinde, wodurch sich die Bruchgefahr der Platte vermindert.
- Die Platte lässt sich mit unterschiedlichen Schrauben bestücken, nämlich solchen, mit denen eine Verblockung zustande kommt und solchen ohne Verblockung. Im letzten Fall fungieren die Schraubenlöcher als neutrale Kompressionslöcher zur Aufnahme des Schraubenkopfes. Dies z.B. beim Applizieren von kleinen Schrauben, um ein Knochenkompartiment zu heften. Hierbei kann die Schraube auch schräg gesetzt werden.
- Da sich die komplette Geometrie der Schraubenlöcher in der Platte von einer Seite bearbeiten lässt, entfällt während der Bearbeitung ein Umspannen, so dass sich insgesamt eine konstengünstige Herstellung ergibt, die ausserdem mit vertretbarem Aufwand präzise ausgeführt werden kann.
- Bei Gestaltung der Knochenplatte mit kreisbogenförmiger Plattenlängsachse
- zumindest im Hauptsegment, falls die Knochenplatte mit einem ein- oder beidseitig daran ansetzenden Lateralsegment versehen ist -, erreicht man nach dem Biegen in den Stegen aus der Plattenebene heraus, quasi über die Fläche, ein Anschmiegen der Knochenplatte an den Knochen. Es entfällt das zusätzliche Biegen in der Plattenebene, quasi das Schränken bzw. Tordieren über die Kante. Durch die kreisbogenförmige Ausgangsgeometrie nimmt die Knochenplatte die Neigung eines zirkulären Abschnittes der Mantelfläche eines Kegels ein. Somit reduzieren sich der operative Aufwand beim Anpassen der Platte und deren Festigkeitsverluste infolge geringerer Verformung. Es ergibt sich eine ideale Anpassung der Knochenplatte in ihrem Längsverlauf und der Neigung über die Plattenbreite.

### Kurzbeschreibung der beigefügten Zeichnungen

Es zeigen:
- Figur 1A -: eine auf einen frakturierten Unterkiefer aufgeschraubte Knochenplatte;
- Figur 1 B -: eine am Unterkiefer aufgeschraubte Knochenplatte zur Überbrückung eines Kontinuitätsdefekts;
- Figur 2A -: eine Knochenplatte mit gleichförmig ausgerichteten Schraubenlöchern in Draufsicht;
- Figur 2B -: die Darstellung gemäss Figur 2A mit verschieden ausgerichteten Schraubenlöchern;
- Figur 2C -: die Knochenplatte gemäss Figur 2A im Schnitt auf der Linie A-A mit der Eingriffskontur *erster Ausführungsform,* Prinzipskizze;
- Figur 2D -: die Darstellung gemäss Figur 2C mit der Eingriffskontur, reale Kontur;
- Figur 2E -: die Knochenplatte gemäss Figur 2A im Schnitt auf der Linie B-B;
- Figur 3A -: eine zweigängige Knochenschraube mit Verblockungsgewinde im Halsbereich;
- Figur 3B -: die zweigängige Knochenschraube gemäss Figur 3A mit Verblockungsgewinde im Halsbereich und dickerem Schraubenschaft;
- Figur 3C -: eine Knochenschraube mit Verblockungsgewinde im Halsbereich und selbstbohrendem sowie selbstschneidendem Gewinde am Schraubenschaft;
- Figur 3D -: die Knochenschraube gemäss Figur 3C mit Verblockungsgewinde im Halsbereich und selbstbohrendem sowie selbstschneidendem Gewinde am Schraubenschaft und zusätzlicher Schnittnut;
- Figur 3E -: eine eingängige selbstschneidende Knochenschraube mit spitzen Gewindeflanken;
- Figur 3F -: eine zweigängige selbstschneidende Knochenschraube mit spitzen Gewindeflanken und dickerem Schraubenschaft;
- Figur 3G -: die zweigängige selbstschneidende Knochenschraube gemäss Figur 3F mit Rundgewinde im Halsbereich;
- Figur 3H -: die eingängige selbstschneidende Knochenschraube gemäss Figur 3E mit spitzen Gewindeflanken, grösserer Steigung und grösserem Aussendurchmesser am Schraubenschaft;
- Figur 4A -: die Knochenschraube gemäss Figur 3A vertikal in ein Schraubenloch aus Figur 2E verblockt eingeschraubt;
- Figur 4B -: die Ansicht gemäss Figur 4A mit schräg verblockt eingeschraubter Knochenschraube;
- Figur 4C -: Knochenschrauben gemäss Figur 3A in Schraubenlöchern mit Eingriffskonturen *erster Ausführungsform* gemäss Figur 2E einer gebogenen Knochenplatte verblockt eingeschraubt;
- Figur 4D -: Knochenschrauben in eine gebogene und tordierte Knochenplatte gemäss Figur 2A eingeschraubt;

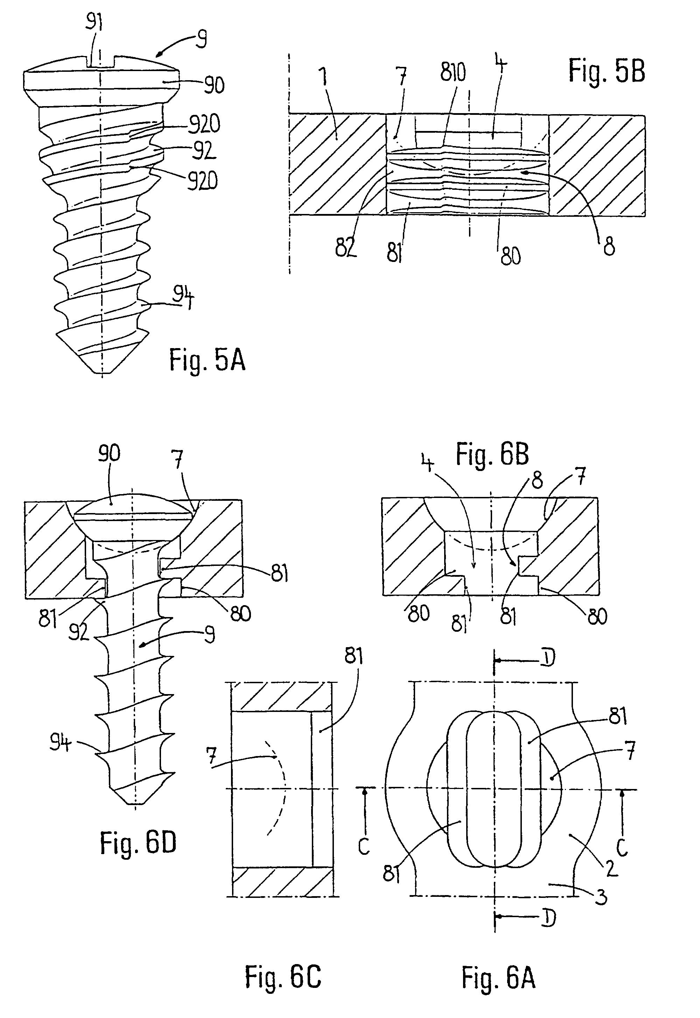

- Figur 5A -: die Knochenschraube gemäss Figur 3A mit Verblockungsgewinde, nach der Verblockung deformiert;
- Figur 5B -: ein Schraubenloch mit einer Eingriffskontur *erster Ausführungsform* aus Figur 2E, nach der Verblockung deformiert;
- Figur 6A -: ein Schraubenloch mit einer Eingriffskontur *zweiter Ausführungsform* in Draufsicht;
- Figur 6B -: die Darstellung gemäss Figur 6A als Schnitt auf der Linie C-C;
- Figur 6C -: die Darstellung gemäss Figur 6A als Schnitt auf der Linie D-D;
- Figur 6D -: die Knochenschraube gemäss Figur 3E in das Schraubenloch aus Figur 6B verblockt eingeschraubt;

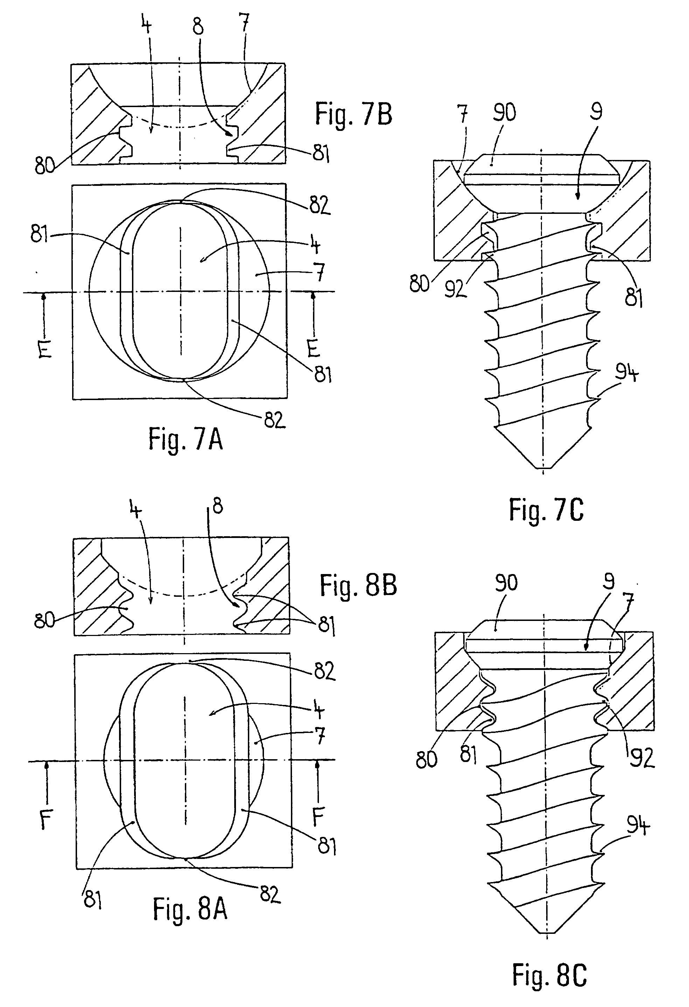

- Figur 7A -: ein Schraubenloch mit einer Eingriffskontur *dritter Ausführungsform* in Draufsicht;
- Figur 7B -: die Darstellung gemäss Figur 7A als Schnitt auf der Linie E-E;
- Figur 7C -: die Knochenschraube gemäss Figur 3F in das Schraubenloch aus Figur 7B verblockt eingeschraubt;
- Figur 8A -: ein Schraubenloch mit einer Eingriffskontur *vierter Ausführungsform* in Draufsicht;
- Figur 8B -: die Darstellung gemäss Figur 8A als Schnitt auf der Linie F-F;
- Figur 8C -: die Knochenschraube gemäss Figur 3G in das Schraubenloch aus Figur 8B verblockt eingeschraubt;
- Figur 9A -: ein Schraubenloch mit einer Eingriffskontur *fünfter Ausführungsform* in Draufsicht;
- Figur 9B -: die Darstellung gemäss Figur 9A als Schnitt auf der Linie G-G;
- Figur 9C -: die Knochenschraube gemäss Figur 3H in das Schraubenloch aus Figur 9B verblockt eingeschraubt;
- Figur 10A -: die Situation vor dem Schliessen einer Fraktur mittels Kompressions-Osteosynthese;
- Figur 10B -: die Situation während des Schliessens der Fraktur;
- Figur 10C -: die Situation nach dem Schliessen der Fraktur;
- Figur 11A -: eine Knochenschraube ohne Verblockungsgewinde;
- Figur 11B -: die Knochenschraube ohne Verblockungsgewinde gemäss Figur 11A mit dickerem Schraubenschaft;
- Figur 11C -: die Knochenschraube gemäss Figur 11A in ein Schraubenloch aus Figur 2E unverblockt und vertikal eingeschraubt;
- Figur 11D -: die Darstellung gemäss Figur 11 C mit unverblockt und schräg eingeschraubter Knochenschraube;
- Figur 11E -: die Situation bei einer Zugschrauben-Osteosynthese mit einer Knochenschraube ohne Verblockungsgewinde gemäss Figur 11A in ein Schraubenloch mit Eingriffskonturen *erster Ausführungsform* gemäss Figur 2E unverblockt eingeschraubt;
- Figur 12A -: die Knochenschraube gemäss Figur 3E in ein Schraubenloch aus Figur 2C unverblockt und vertikal eingeschraubt;
- Figur 12B -: die Darstellung gemäss Figur 12A mit unverblockt und schräg eingeschraubter Knochenschraube;

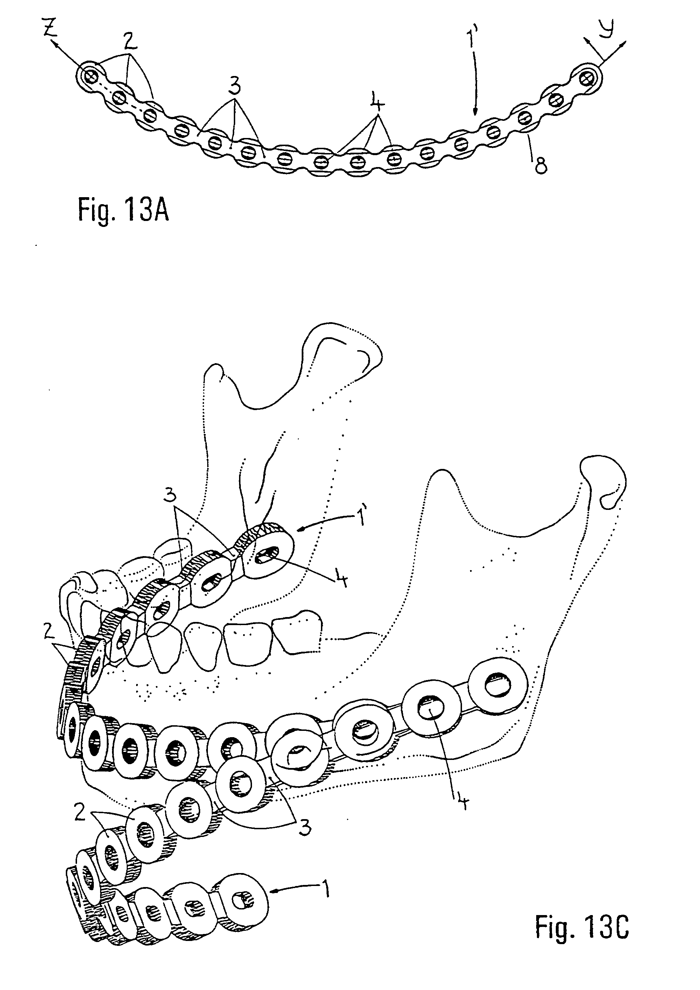

- Figur 13A -: eine Knochenplatte mit kreisbogenförmiger Plattenlängsachse Z und 16 Schraubenlöchern;
- Figur 13B -: eine vergrösserte Darstellung von drei Plattengliedern gemäss Figur 13A;
- Figur 13C -: eine Prinzipdarstellung eines menschlichen Unterkiefers mit der sich anpassenden kreisbogenförmigen Knochenplatte gemäss Figur 13A und einer geraden, vom Unterkiefer abdriftenden Knochenplatte gemäss Figur 2A;
- Figur 14A -: eine Knochenplatte für den linken Kieferast mit einem kreisbogenförmigen Hauptsegment gemäss Figur 13A, einem geraden, aufsteigendem Lateralsegment und verstärktem Winkelbereich mit einem zusätzlichen Schraubenloch;
- Figur 14B -: die Knochenplatte gemäss Figur 14A mit gebogenem Lateralsegment und verstärktem Winkelbereich ohne zusätzliches Schraubenloch;
- Figur 14C -: die Knochenplatte gemäss Figur 14B mit zusätzlichem Schraubenloch im verstärkten Winkelbereich;
- Figur 14D -: die Knochenplatte gemäss Figur 14A ohne zusätzliches Schraubenloch im verstärkten Winkelbereich; und
- Figur 14E -: die Knochenplatte gemäss Figur 14A mit verringertem Abstand der Schraubenlöcher im Lateralsegment.

### Ausführungsbeispiele

Mit Bezug auf die beiliegenden Zeichnungen erfolgt nachstehend die detaillierte Beschreibung von Ausführungsbeispielen zur erfindungsgemässen osteosynthetischen Knochenplatte mit verblockbaren Knochenschrauben.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen. Im Interesse der Übersichtlichkeit wird auf die wiederholte Bezeichnung von Bauteilen in weiteren Figuren zumeist verzichtet, sofern zeichnerisch eindeutig erkennbar ist, dass es sich um "wiederkehrende" Bauteile handelt.

### Figuren 1A und 1B

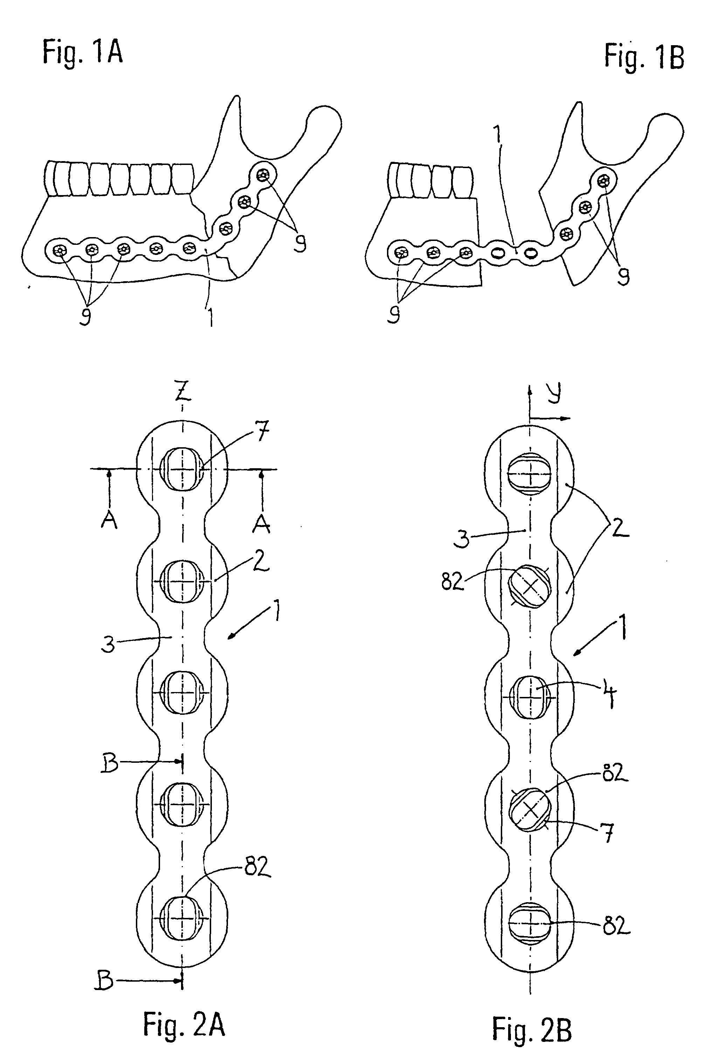

Diese Figuren zeigen zwei typische Anwendungsfälle der erfindungsgemässen verblockbaren Knochenplatte 1, welche zusammen mit Knochenschrauben 9 eine Platten-Schrauben-Verbindung ergibt. Zum einen ist die Platte 1 vorrangig zur Osteosynthese eines in Knochenkompartimente fraktuierten Unterkiefers geeignet (s. Figur 1A). Eine weitere hauptsächliche Anwendung liegt in der Überbrückung eines Kontinuitätsdefekts, d.h. bei einem fehlenden Knochenstück muss dauerhaft durch die Platte 1 die Belastung aufgenommen und Stabilität am Unterkiefer hergestellt werden (s. Figur 1 B). Zu vermeiden sind sowohl das Aufpressen der Platte 1 auf die Knochenhaut und ein unbeabsichtigtes Lösen der eingedrehten Schrauben, so hier eine solide Verblockung besonders relevant ist.

### Figuren 2A bis 2E

In der einfachsten Form ist die Platte **1** ein langgestreckter unverzweigter Ast, durch den sich die Plattenlängsachse **Z** erstreckt. Die Platte **1** setzt sich aus einer Vielzahl von Plattengliedem **2** zusammen, die jeweils durch Stege **3** miteinander verbunden sind. Die Stege **3** bilden taillierte Übergänge zwischen benachbarten Plattengliedern. Zumindest in einigen Plattengliedern **2,** vorzugsweise jedoch in jedem Plattenglied, ist ein Schraubenloch **4** vorhanden. Auf der Plattenoberseite **5** sind die auf der Plattenunterseite **6** austretenden Schraubenlöcher **4** von einer kugelförmigen Ansenkung **7** umgeben. Innerlich im Schraubenloch **4** befindet sich eine Eingriffskontur **8**.

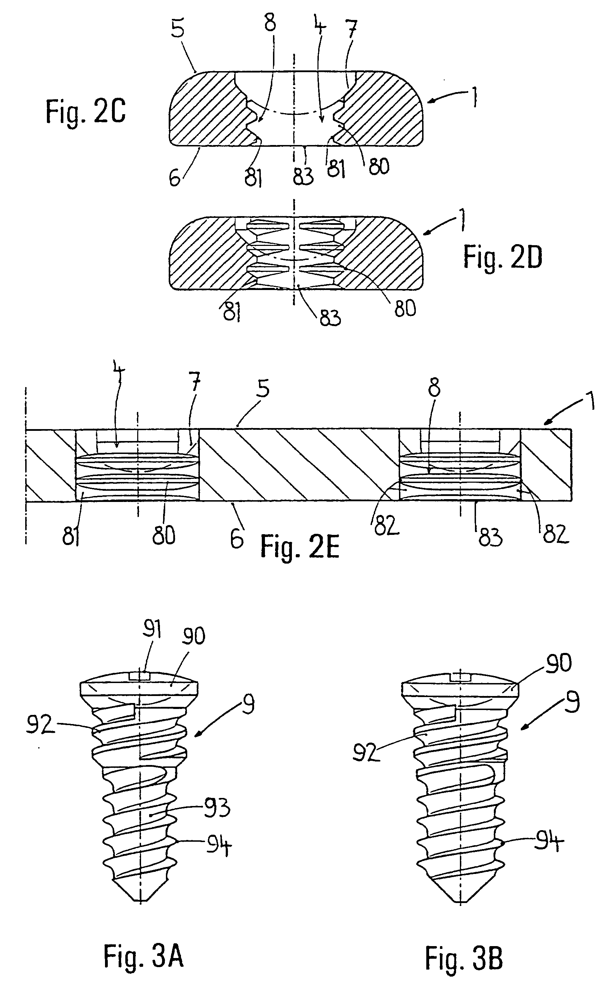

Die Eingriffskontur **8** gleicht nur auf den ersten Blick einem partiellen Gewinde; tatsächlich besteht die Eingriffskontur **8** aus an der Wandung des Schraubenlochs **4** parallel zur Plattenebene **Y** erstreckten Konturtälern **80** und dazu alternierenden benachbarten Konturspitzen **81.** Die Konturtäler **80** und -spitzen **81** laufen partiell im Schraubenloch **4** um, also zu den Rändern der Eingriffskontur **8** aus, so dass dort unkonturierte Wandungsbereiche **82** im Schraubenloch **4** verbleiben. Die Ansenkung **7** hat eine Tiefe, um die Schraubenköpfe versenkt aufzunehmen. Der Durchmesser der Ansenkung **7** ist grösser als die lichte Weite der Eingriffskontur **8.** Die Distanz zwischen gegenüber liegenden Konturtälern **80** und Konturspitzen **81** ist geringer als die Distanz zwischen den gegenüber liegenden unkonturierten Wandungsbereichen **82**, so dass sich der Austritt **83** des Schraubenlochs **4** an der Plattenunterseite **6** langlochförmig zeigt. Die eine Eingriffskontur **8** bildenden Konturtäler **80** und Konturspitzen **81** können im wesentlichen parallel zur Plattenlängsachse Z oder im Winkel zu dieser angeordnet sein, so dass die Längserstreckung des Austritts **83** auf der Plattenlängsachse **Z** liegt (s. Figur 2A) oder verschiedene Winkel zur Plattenlängsachse **Z** einnimmt (s. Figur 2B). Entsprechend verteilen sich die unkonturierten Wandungsbereiche **82** entlang der Plattenlängsachse **Z** oder zu dieser versetzt. In der hiesigen *ersten Ausführungsform* der Eingriffskontur **8** bilden die Konturtäler **80** und Konturspitzen **81** trapezförmige Gänge.

### Figur 3A

Die Knochenschraube **9** besitzt zuoberst den Schraubenkopf **90,** der unten ka-Iottenförmig, komplementär zur Ansenkung **7** in der Platte **1** ausgebildet ist. Im Schraubenkopf **90** ist eine von oben mit einem Eindrehwerkzeug fassbare Ausnehmung **91**, z.B. ein Kreuzschlitz, vorhanden. Unterhalb des Schraubenkopfes **90** befindet sich ein Verblockungsgewinde **92,** hier als Trapezgewinde - korrespondierend zur Eingriffskontur **8** - gestaltet. Längs des Schraubenschafts **93** ist das zweigängige Knochengewinde **94** angeordnet. Das Knochengewinde **94** hat z.B. einen Aussendurchmesser von 2.5 mm, während der Aussendurchmesser des Verblockungsgewindes **92** deutlich grösser ist und z.B. 3.2 mm beträgt.

### Figur 3B

Ist der Durchmesser von z.B. 2.5 mm am Knochengewinde **94** gemäss der vorherigen Schraube **9** zu gering, da das Schraubenloch im Knochen zu gross gebohrt wurde oder die Festigkeit nicht ausreicht, kann man eine modifizierte Schraube **9** mit grösserem Durchmesser am Knochengewinde **94** einsetzen, der z.B. 3.2 mm beträgt. Auch bei diesem Durchmesser lässt sich das Knochengewinde 94 mühelos durch die Eingriffskontur **8** in der Platte **1** bewegen. Die breiten trapezförmigen Flanken des Verblockungsgewindes **92** hingegen führen zu einer Verblockung mit der Eingriffskontur **8.**

### Figuren 3C und 3D

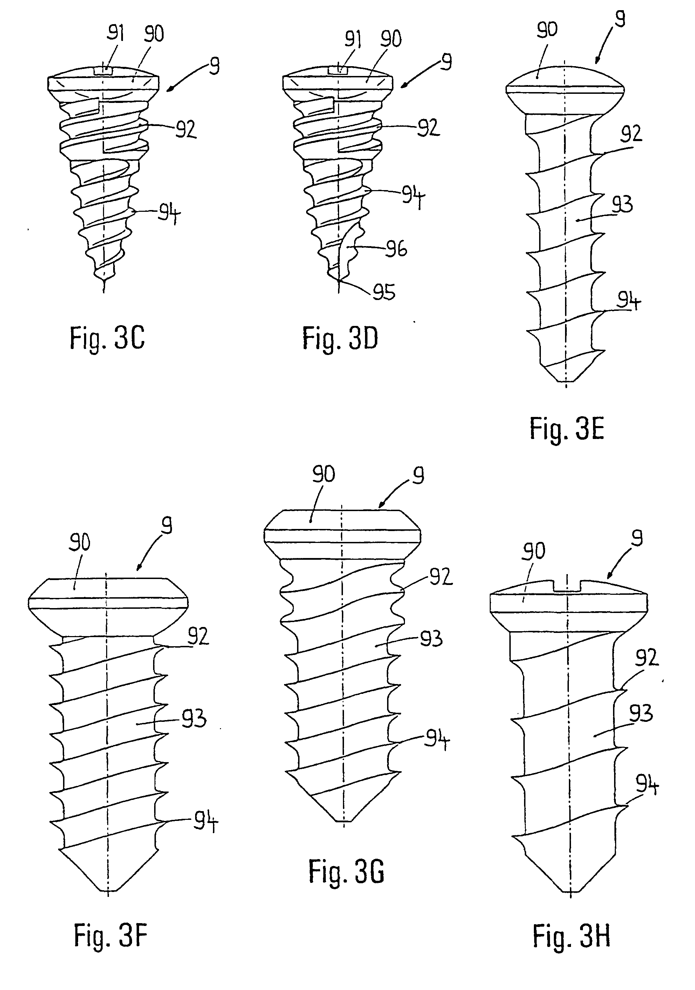

Für eine verblockbare Platten-Schrauben-Verbindung kann alternativ die gezeigte selbstbohrende Schraube **9** verwendet werden, welche ebenfalls unterhalb des Schraubenkopfes **90** ein trapezförmiges Verblockungsgewinde **92** besitzt, von dem sich das Knochengewinde **94** erstreckt. Beispielhaft weisen das Knochengewinde **94** einen maximalen Aussendurchmesser von 2.5 mm und das Verblockungsgewinde **92** einen Aussendurchmesser von 3.2 mm auf. Die Schraube **9** gemäss Figur 3D ist an der Schaftspitze **95** zusätzlich mit einer Schnittnut **96** versehen.

### Figuren 3E, 3F und 3H

Diese Schrauben **9** besitzen ein über den Schraubenschaft **93** durchgängig einheitliches Gewinde mit spitzen Gewindeflanken, das mit den passend konturierten Eingriffskonturen **8** in den Platten **1** im oberen Teil als Verblockungsgewinde **92** und im unteren Teil als Knochengewinde **94** zum Einsatz kommt. Die Schrauben **9** gemäss den Figuren 3E und 3H sind eingängig, die Schraube **9** zu Figur 3F ist zweigängig.

### Figur 3G

Hier ist eine zweigängige Schraube **9** mit runden Gewindeflanken am Verblockungsgewinde **92** und spitzen Gewindeflanken am Knochengewinde **94** gezeigt.

### Figuren 4A bis 4D

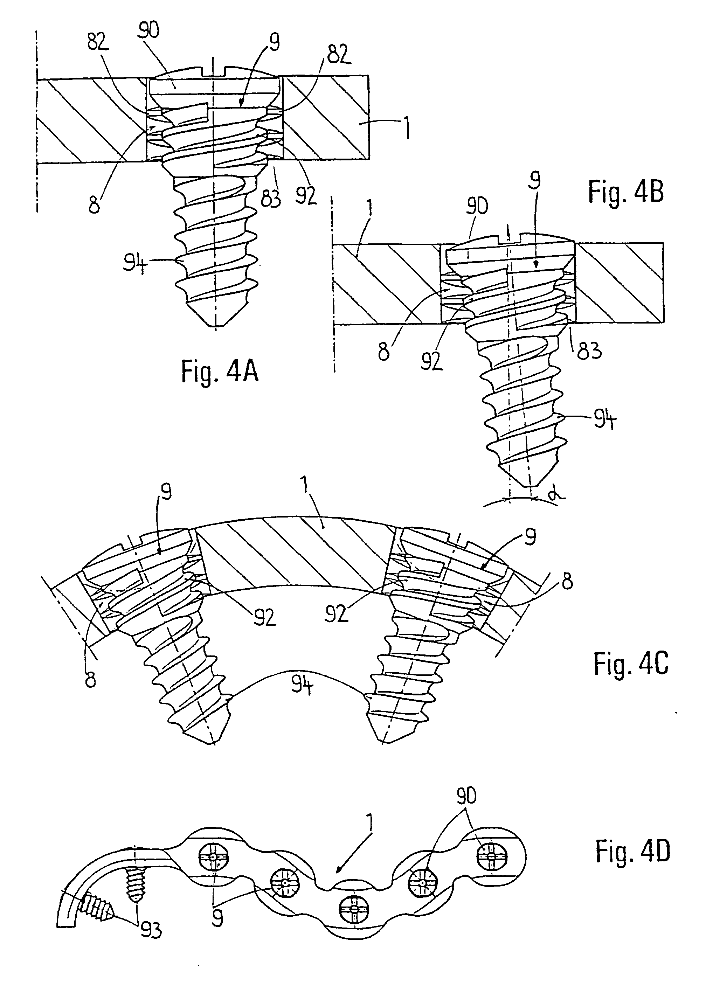

Die Eingriffskontur **8** in der Platte **1** erlaubt, die Schraube **9** mit dem Verblockungsgewinde **92** sowohl senkrecht (s. Figur 4A) als auch mit Winkelversatz α im Verhältnis zur Plattenlängsachse **Z** (s. Figur 4B) in das Schraubenloch **4** einzudrehen. Auch bei schräger Positionierung der Schraube **9** kommt eine voll wirksame Verblockung zustande; das Verblockungsgewinde **92** fährt ebenfalls in die Eingriffskontur **8** ein. Die an den Enden der Eingriffskontur **8** liegenden unkonturierten Wandungsbereiche **82** mit der Längserstreckung des Austritts **83** bieten Freiraum für eine Annäherung des schräg stehenden Verblockungsgewindes **92**. Durch die Schrägstellung der Schraube **9** kommt der Schraubenkopf **90** angeschrägt in der Ansenkung **7** zu liegen. Die praktisch volle und fugenlose Auflage des Schraubenkopfes **90** in der Ansenkung **7** ergibt sich durch die aufeinander liegenden komplementären kugeligen Oberflächen.

Ein Biegen der Platte **1** um die Plattenebene **Y** (s. Figur 4C) bzw. in der Plattenebene **Y** oder das Tordieren der Platte **2** (s. Figur 4D) beeinträchtigen die Funktionsfähigkeit der Verblockung nicht. Einerseits geschieht die wesentliche Formänderung der Platte **1** im Bereich der Stege **3** und andererseits verliert die Eingriffskontur **8** durch eine begrenzte Deformation nicht an Wirksamkeit.

### Figuren 5A und 5B

Hier wird die bei der Verblockung zwischen der Eingriffskontur **8** und dem in letztere einfahrenden Verblockungsgewinde **92** hervorgerufene Deformation gezeigt, welche in sehr wirksamer Weise die Schraube 9 gegen ungewolltes Lösen sichert. Eingriffskontur **8** und Verblockungsgewinde **92** sind zueinander nicht komplementär. Das Verblockungsgewinde **92** besitzt einen sich wendelförmig fortsetzenden Gewindezahn; bei einem zweigängigen Gewinde entsprechend zwei Zähne. Die Eingriffskontur **8** hingegen besteht aus den Konturtälern **80** und Konturspitzen **81**, welche nicht in einem Steigungswinkel, wie bei einem Gewinde als Wendel verlaufen. Beim Einfahren des Verblockungsgewindes **92** zwischen die Konturspitzen **81** kommt es zu einer gewollten Kollision und gegenseitigen Deformation. Das Verblockungsgewinde **92** verlangt als Ergänzung an sich wendelförmige Innengewindegänge und die horizontal verlaufenden Konturspitzen **81** stemmen sich gegen das einfahrende Verblockungsgewinde **92.** Durch diesen Gegensatz deformieren sich das Verblockungsgewinde **92** und die Eingriffskontur **8**, d.h. an beiden entstehen gegeneinander gerichtete Biegekanten **920,810**, die beim Ausdrehen der Schraube **9** aufeinander stossen und somit einen erheblichen Widerstand gegen ungewolltes Lösen bilden. Beim Ausdrehen der Schraube **9** muss man ein starkes Drehmoment ausüben, um eine teilweise Rückverformung der Biegekanten **920,810** zu erreichen und den erhöhten Reibwiderstand zu überwinden. Für die Eingriffskontur **8** liegt der Vorzugsbereich des vertikalen Abstandes zwischen sich wiederholenden Strukturen - den Konturtälern **80** oder den Konturspitzen **81** - zwischen 0.5 mm und 1.0 mm. Die Konturtäler **80** lassen sich zweckmässig durch Fräsen herstellen, so dass die Konturspitzen **81** zwischen den eingefrästen Konturtälern **80** verbleiben.

### Figuren 6A bis 6D

Die *zweite Ausführungsform* der Eingriffskontur **8** wird ebenfalls aus Konturtälern **80** und Konturspitzen **81** gebildet, die parallel zur Plattenlängsachse **Z** in die Wandung des Schraubenlochs **4** eingearbeitet sind und an ihren Enden in unkonturierten Wandungsbereichen **82** auslaufen. Die Besonderheit besteht hier darin, dass sich beidseits der Plattenlängsachse **Z** Konturtäler **80** und Konturspitzen **81** zueinander versetzt gegenüber liegen. Auf diese Weise wirkt das am Schraubenschaft **93** an sich einheitliche Gewinde im oberen Teil als Verblockungsgewinde **92,** dessen Gewindezähne die Konturspitzen **81** hintergreifen, während der untere Teil als Knochengewinde **94** zum Eindrehen in den Knochen dient. Der unten kalottenförmige Schraubenkopf **90** sitzt in der kugelförmigen Ansenkung **7.**

### Figuren 7A bis 7C

Die *dritte Ausführungsform* der Eingriffskontur **8** setzt sich aus Konturtälern **80** und Konturspitzen **81** zusammen, die sich innerhalb des Schraubenlochs **4** parallel zur Plattenlängsachse **Z** erstrecken und sägenförmig sind. Hier liegen die Konturtäler **80** und Konturspitzen **81** beidseits der Plattenlängsachse **Z** auf gleicher Höhe und laufen an den Enden der Eingriffskontur **8** in unkonturierten Wandungsbereichen **82** aus. Auch die in der hiesigen Platten-Schrauben-Verbindung eingesetzte zweigängige Schraube **9** weist über ihren Schraubenschaft **93** ein gleichförmiges Gewinde auf, dessen oberer Teil als Verblockungsgewinde **92** die Konturspitzen **81** untergreift und dessen unterer Teil als Knochengewinde **94** zum Eindrehen in den Knochen vorgesehen ist. Der Schraubenkopf **90** wird wiederum in der kugelförmigen Ansenkung **7** eingebettet.

### Figuren 8A bis 8C

Bei dieser *vierten Ausführungsform* der Eingriffskontur **8** haben die Konturtäler **80** und die Konturspitzen **81** rundliche Form. Komplementär zur rundlichen Form der Eingriffskontur **8** wird eine Schraube **9** mit einem zweigängigen Verblockungsgewinde 92, das als Rundgewinde ausgebildet ist, verwendet. Unterhalb des Verblockungsgewindes **92** erstreckt sich das spitze, zweigängige Knochengewinde **94.** Die rundlichen Konturtäler **80** und Konturspitzen **81** liegen beidseits der Plattenlängsachse **Z** auf gleicher Höhe mit an den Enden der Eingriffskontur **8** unkonturierten Wandungsbereichen **82.** Das Verblockungsgewinde **92** kommt mit den Konturtälern **80** und -spitzen **80,81** in verblockten Eingriff. Der Schraubenkopf **90** findet in der Ansenkung **7** Platz.

### Figuren 9A bis 9C

Die Besonderheit bei der *fünften Ausführungsform* der Eingriffskontur **8** besteht darin, dass die Konturspitzen **81** im Schraubenloch **4** zueinander in der Plattenebene **Y** um jeweils 120° zueinander versetzt sind und in der Höhe - bezogen auf die Plattenebene **Y** - treppenförmig zueinander liegen. Konturtäler und - spitzen **80,81** haben eckige Form. Korrespondierend zur Eingriffskontur **8** hat die verwendete Schraube **9** ein über ihren Schraubenschaft **93** verlaufendes gleichförmiges Gewinde, dessen oberer Teil als Verblockungsgewinde **92** die Konturspitzen **81** untergreift und dessen unterer Teil das Knochengewinde **94** darstellt. Dem Schraubenkopf **90** bietet die kugelförmige Ansenkung **7** Platz.

### Figuren 10A bis 10C

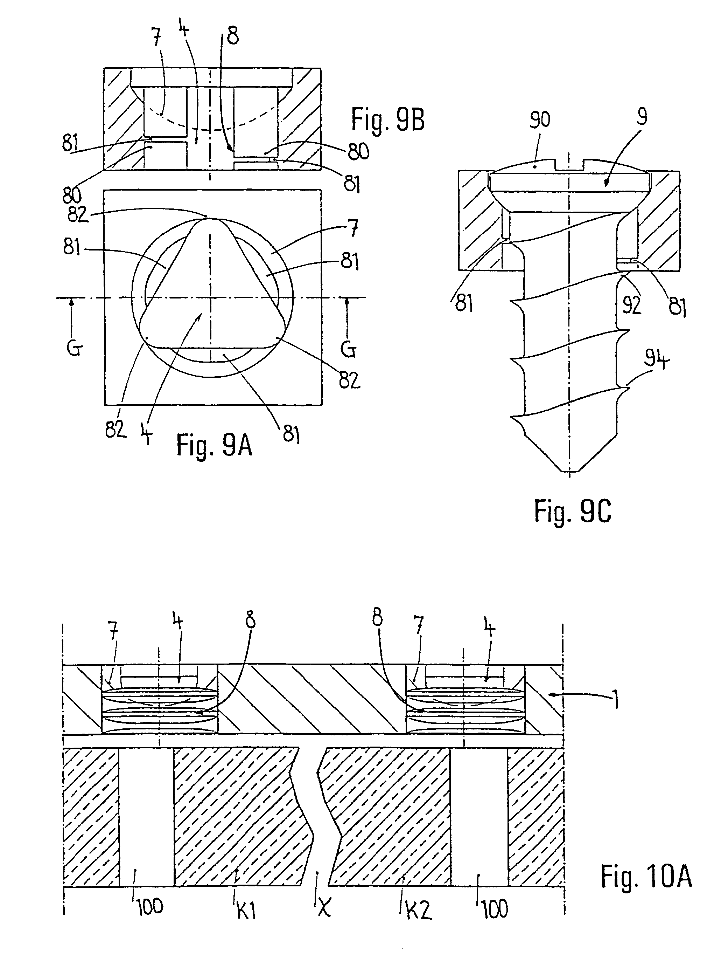

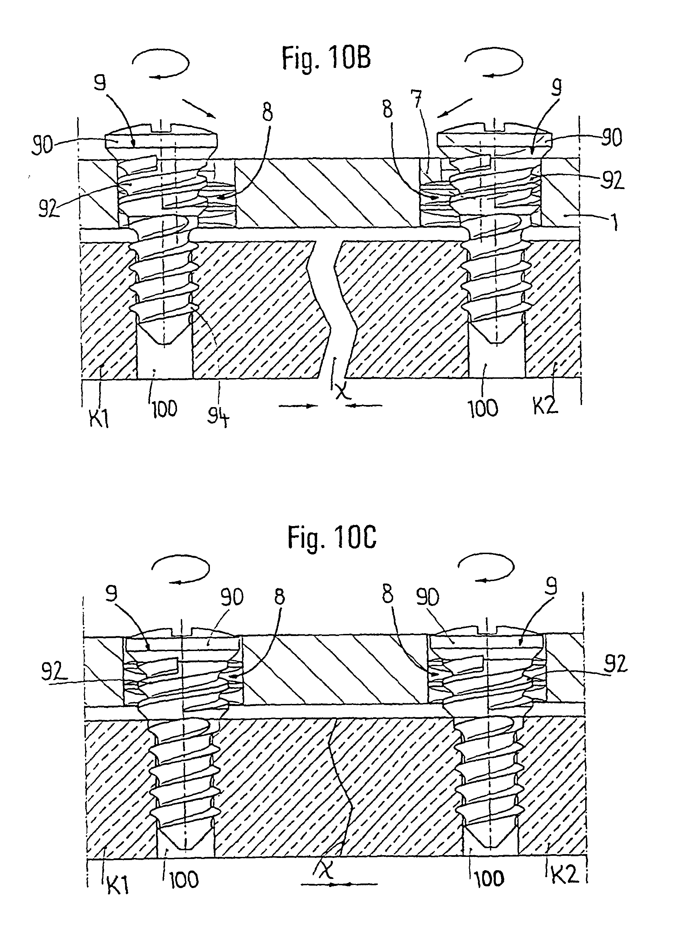

Am Beispiel einer Kompressions-Osteosynthese wird die klinische Applikation der Platten-Schrauben-Verbindung gezeigt. In der Ausgangssituation (s. Figur 10A), also vor dem Schliessen der Fraktur, werden in den beiden zu verbindenden Knochenkompartimenten **K1,K2** - im Verhältnis zu den Schraubenlöchem **4** in der Platte **1** - exzentrische Bohrungen **100** angebracht. Der Abstand zwischen den Bohrungen **100** ist grösser als der Abstand zwischen den Schraubenlöchern **4** in der Platte **1,** welche über der Bruchstelle mit dem Knochenspalt **X** in Position gebracht wird. Die Schraubenlöcher **4** weisen die Eingriffskonturen **8** und die Ansenkungen **7** auf.

Zum Schliessen der Fraktur (s. Figur 10B) werden durch die Schraubenlöcher **4** der Platte **1** hindurch Schrauben **9** in die Bohrungen **100** eingebracht. Zunächst, noch mit überstehenden Schraubenköpfen **90**, stehen die Schrauben **9** exzentrisch in den Schraubenlöchern **4.** Mit tieferem Eindrehen der Schrauben **9** dringt das Knochengewinde **94** tiefer in die Bohrungen **100** ein und die Kugelflächen unten am Schraubenkopf **90**, im Zusammenwirken mit den kalottenförmigen Ansenkungen **7** in den Schraubenlöchern **4,** bewirken die sukzessive Zentrierung der Schrauben **9**. Zugleich beginnt das Verblockungsgewinde **92** der Schrauben **9** in die Eingriffskontur **8** einzufahren. Bei der Zentrierung der Schrauben **9** werden diese aufeinanderzu bewegt und nehmen dabei die Knochenkompartimente **K1,K2** mit; der Knochenspalt **X** beginnt sich zu schliessen.

In der Endstellung (s. Figur 10C) sind die Schraubenköpfe **90** maximal in den Ansenkungen **7** eingebettet und die Schrauben **9** soweit zentriert, dass der Knochenspalt **X** völlig geschlossen ist. Die Knochenkompartimente **K1,K2** werden nun aneinander gepresst. Zwischen dem Verblockungsgewinde **92** der Schrauben **9** und den Eingriffskonturen **8** ist die Verblockung hergestellt; die Schrauben **9** sind somit gegen selbständiges Lösen gesichert.

### Figuren 11 A bis 11D

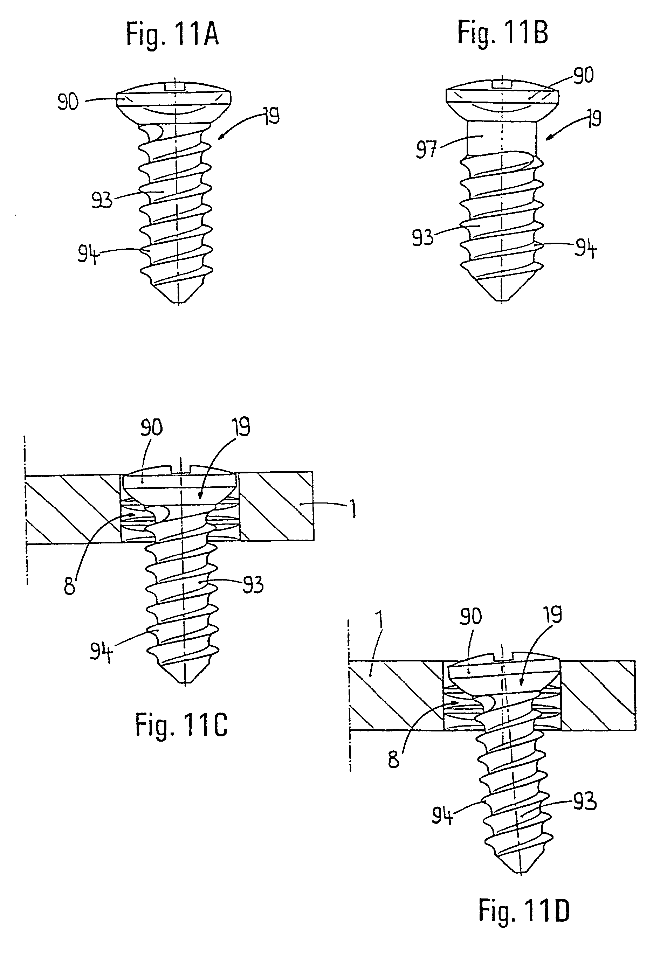

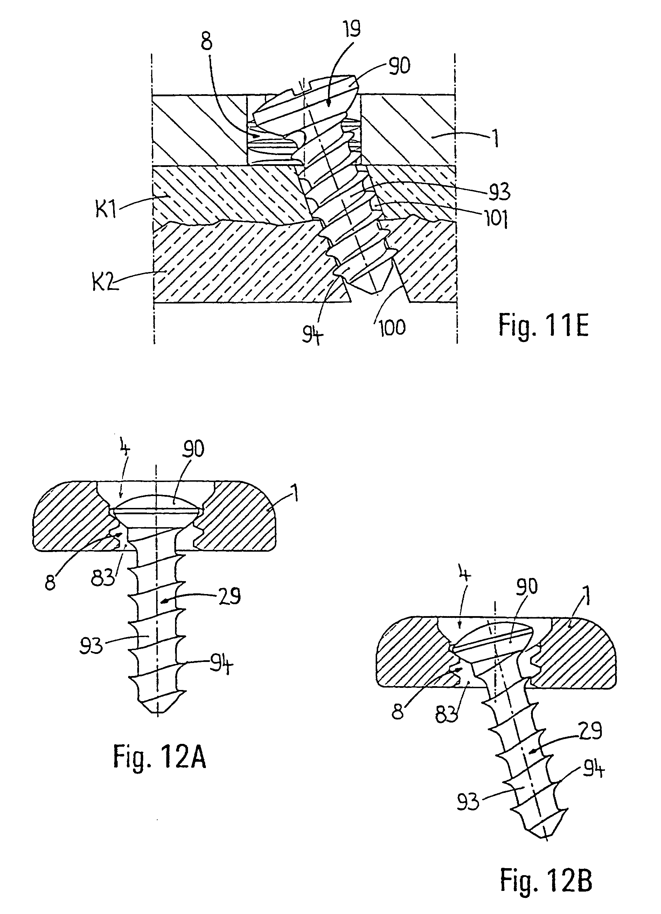

Die zwei gezeigten Schrauben **19** (s. Figuren 11A und 11B) besitzen kein Verblockungsgewinde **92.** Die Schraube **19** aus Figur 11A, z.B. mit einem Aussengewindedurchmesser von 2.5 mm, ist zu schwach, um mit der Eingriffskontur **8** in Verblockung zu kommen. Bei der Schraube **19** aus Figur 11B mit einem stärkeren Aussengewindedurchmesser von z.B. 3.0 mm ist unterhalb des Schraubenkopfes **90** ein Hinterstich **97** vorgesehen, so dass auch hier kein Verblockungsgewinde **92** vorhanden ist. Diese Schrauben **19** ohne Verblockungsgewinde **92**, also nur mit dem Knochengewinde **94**, können ebenfalls zusammen mit der Platte **1,** welche in ihren Schraubenlöchern Eingriffskonturen **8** aufweist, verwendet werden. Eine Verblockung kommt hier, wie bei der konkreten Anwendung eventuell gewünscht, nicht zustande (s. Figur 11C). Ohne weiteres lässt sich die Schraube **19** ohne Verblockungsgewinde **92** auch schräg eingesetzt zusammen mit der Platte **1** applizieren (s. Figur 11D).

### Figur 11E

Diese Darstellung zeigt eine Zugschrauben-Osteosynthese mit der erfindungsgemässen Platte **1** und einer herkömmlichen Schraube **19** ohne Verblockungsgewinde **92.** Der Schraubenkopf **90** stützt sich schräg im Schraubenloch **4** ab und der Schraubenschaft **93** durchragt die beiden Knochenkompartimente **K1,K2,** welche aufeinander gepresst werden sollen. Im oberen Knochenkompartiment **K1** ist eine Durchgangsbohrung **101** der Weite vorhanden, dass das Knochengewinde **94** nicht greifen kann. Im unteren Knochenkompartiment **K2** hat man eine im Durchmesser verminderte Bohrung **100** vorgesehen, wo das Knochengewinde **94** eingreift. Mit dem Eindrehen der Schraube **19** wird das untere Knochenkompartiment **K2** an das obere Knochenkompartiment **K1** heran gezogen. Die Eingriffskontur **8** kommt hier nicht zur Funktion; der dünne Schraubenschaft **93** durchragt die Eingriffskontur **8** ohne eine Verblockung einzugehen.

### Figuren 12A und 12B

Die beiden Figuren sollen der Vollständigkeit halber veranschaulichen, dass in die Platte **1** auch Kleinfragmentschrauben **29**, z.B. mit einem Aussengewindedurchmesser von 2.0 mm, vertikal oder schräg eingesetzt werden können. Der kleine Schraubenkopf **90** stützt sich tiefliegend im Schraubenloch **4** ab und die Eingriffskontur **8** bleibt ohne Funktion. Solche Kleinfragmentschrauben **29** mit einem aus dem Austritt **83** herausragenden Schraubenschaft **93** und dem Knochengewinde **94** dienen zum Anheften von kleineren Knochenfragmenten.

### Figuren 13A und 13B

Diese Knochenplatte **1'** besitzt eine kreisbogenförmige Plattenlängsachse **Z** mit einer Vielzahl von Plattengliedern **2,** die durch Stege **3** miteinander verbunden sind. Die Platte **1'** erstreckt sich im ungebogenen Ausgangszustand in der Plattenebene **Y.** In den Plattengliedern **2** ist jeweils ein Schraubenloch **4** mit der zuvor beschriebenen speziellen Eingriffskontur **8** für die Verblockung vorgesehen. Durch die kreisbogenförmige Plattenlängsachse **Z** ergibt sich zwischen den Mittelachsen **Z**_{**1**} der Stege **3** und der Mittelachse **Z**_{**2**} des jeweils benachbarten Schraubenlochs **4** eine Versetzung β mit z.B. 1° ≤ β ≤ 10° und vorzugsweise β = 2.5°. Zwischen zwei benachbarten Schraubenlöchern **4** ergibt sich eine Versetzung von 2β mit z.B. 2° ≤ 2β ≤ 20° und vorzugsweise 2β = 5°. Es ist nicht zwingend, dass alle Schraubenlöcher **4** für die Verblockung die Eingriffskontur **8** aufweisen.

An der Platte **1'** können auch zylindrische Standard-Schraubenlöcher oder richtungsorientierte Kompressionslöcher vorhanden sein. Vorzugsweise erhält die kreisbogenförmige Platte **1'** ihre Gestalt ohne Umformen. Je nach Verwendungszweck wird die Platte **1'** in der erforderlichen Länge hergestellt bzw. durch Abtrennen von Plattengliedern **2** und Stegen **3** auf die gewünschte Länge gebracht. Ebenso abhängig vom Verwendungszweck können die Anzahl, die Positionierung, der Abstand und die Art der Schraubenlöcher **4** gewählt werden.

### Figur 13C

Diese Figur veranschaulicht als Prinzipdarstellung die Erstreckungsrichtung und die geometrische Anpassung einer Platte **1** mit gerader Plattenlängsachse **Z** bzw. einer Platte **1'** mit kreisbogenförmig verlaufender Plattenlängsachse **Z,** wobei beide Platten **1,1'** an einem menschlichen Unterkiefer angesetzt sind und sich im fertig angebogenen Zustand, den Unterkiefer umfassend, zwischen den aufsteigenden Kieferästen erstrecken.

Die am linken Kieferast angesetzte gerade Platte 1 driftet nach dem Biegen in den Stegen **3** aus der Plattenebene **Y** heraus - quasi über die Fläche - vom Unterkiefer ab. Damit diese Platte **1** den Unterkiefer umfasst, muss ein zusätzliches Biegen in der Plattenebene **Y** in jedem Steg **3** - quasi über die Kante - vorgenommen werden. Erst hiemach folgt die Platte 1 dem Verlauf des Unterkiefers. Da dieser im Prinzip keine vertikalen Flächen aufweist, sondern sich nach apikal vorstreckt, ist - um ein ordentliches Anschmiegen der Platte **1** an den Kieferknochen zu erreichen -, ein nachträgliches Schränken bzw. Tordieren der Plattenglieder **2** erforderlich.

Viel günstiger wird das Anschmiegen der Platte 1' an den Kieferknochen bei kreisbogenförmig verlaufender Plattenlängsachse **Z** erreicht. Es muss lediglich ein Biegen der Platte **1'** in den Stegen **3** aus der Plattenebene **Y** heraus, quasi über die Fläche, erfolgen, um die Platte in die den Unterkiefer umfassende U-Form zu bringen. Das Biegen geschieht in aller Regel im wesentlichen im Bereich der Stege **3.** Das zusätzliche Biegen in der Plattenebene **Y,** quasi über die Kante, sowie das Schränken bzw. Tordieren entfallen hier.

Aufgrund der kreisbogenförmigen Ausgangsgeometrie nimmt die Platte **1'**, nach dem Biegen aus der Plattenebene **Y** heraus, die Neigung eines zirkulären Abschnittes der Mantelfläche eines Kegels ein. Damit sind sowohl das im Prinzip horizontale, U-förmige Umfassen des Unterkiefers im Längsverlauf der Platte **1'** als auch das enge Anschmiegen der Platte **1'** über deren Breite an den apikal vorgestreckten Knochen realisiert. Durch die kreisbogenförmige Ausgangsgeometrie vermindert sich der operative Aufwand beim Anpassen der Platte **1'** wesentlich, die Platte **1'** schmiegt sich ideal an den Unterkiefer an und die Festigkeitsverluste werden infolge geringerer Verformung - nur aus der Plattenebene **Y** heraus - reduziert. Die positiven Effekte treten besonders dann auf, wenn die kreisbogenförmige Platte **1'** ohne Umformen hergestellt wird, so dass die Platten **1'** in der bogenförmigen Geometrie aber ohne vorheriges Biegen zum Chirurgen gelangen.

### Figuren 14A bis 14E

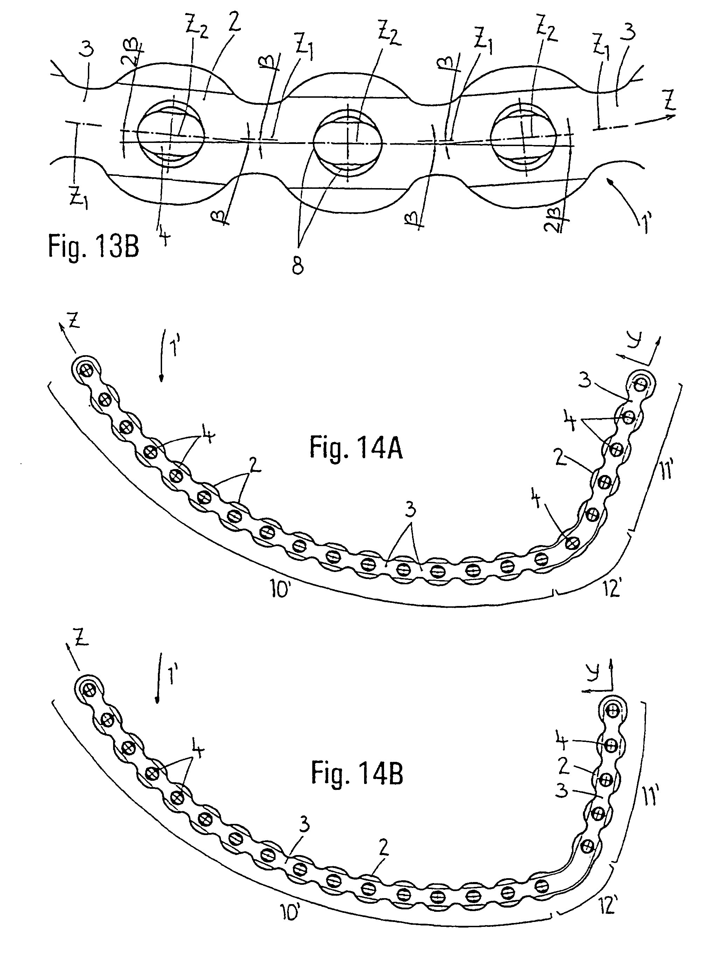

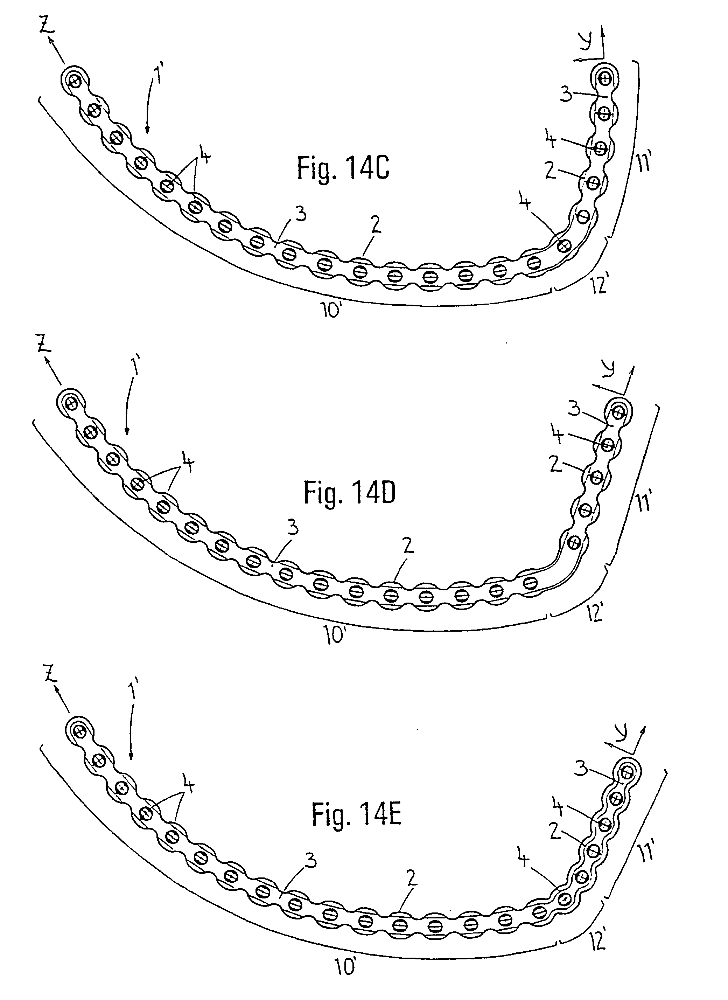

In dieser Figurenserie werden verschieden modifizierte Platten **1'** mit kreisbogenförmig verlaufender Plattenlängsachse **Z** gezeigt. Sämtliche Platten **1'** besitzen ein kreisbogenförmiges Hauptsegment **10'**, an das verlängernd rechts oder links oder beidseits ein Lateralsegment **11'** ansetzt. Das Hauptsegment **10'** ist hier z.B. einheitlich mit 16 zur Verblockung geeigneten Schraubenlöchern **4** vorgesehen. Im Übergang zwischen dem Hauptsegment **10'** und dem Lateralsegment **11'** befindet sich ein Winkelsegment **12'**, das im Verhältnis zu den Stegen **3** vorzugsweise in der Breite verstärkt ist. Das Winkelsegment **12'** kann lochfrei sein (s. Figuren 14B und 14D) oder zumindest ein Schraubenloch **4** aufweisen (s. Figuren 14A, 14C und 14E). Das Lateralsegment **11'** kann sich in der Plattenebene **Y** gerade (s. Figuren 14A, 14D und 14E) oder gekrümmt (s. Figuren 14B und 14C) erstrecken. Die Modifikation gemäss Figur 14E zeigt eine Platte **1'** mit kreisbogenförmigem Hauptsegment **10'** und einem geraden Lateralsegment **11'** mit verringertem Abstand der **5** gezeigten Schraubenlöcher **4**, wobei das dazwischen liegende Winkelsegment **12'** ein Schraubenloch **4** aufweist. Sofern ein- oder beidseitig des kreisbogenförmigen Hauptsegments **10'** ein gebogenes Lateralsegment **11'** vorgesehen ist, kann letzteres einen vom Hauptsegment **10'** verschiedenen Biegeradius aufweisen.

## Patentansprüche

1. Verblockbare Knochenplatte **(1,1')** mit:
a) mehreren Plattengliedern **(2),** die durch taillenförmige Stege **(3)** miteinander verbunden sind und im ungebogenen Zustand auf einer Plattenebene **(Y)** liegen;
b) zumindest in einigen Plattengliedern **(2)** jeweils einem Schraubenloch **(4);**
c) durch die Schraubenlöcher **(4)** einführbaren Knochenschrauben **(9)** zur Befestigung der Knochenplatte **(1,1');** wobei
d) die einzelne Knochenschraube **(9)** einen im Durchmesser verdickten Schraubenkopf **(90),** ein darunter gelegenes Verblockungsgewinde **(92)** und ein Knochengewinde **(94)** aufweist, das sich zumindest anteilig über den Schraubenschaft **(93)** erstreckt, welcher einen geringeren Querschnittsdurchmesser als der Schraubenkopf **(90)** besitzt;
e) innerhalb des einzelnen Schraubenlochs **(4)** eine Eingriffskontur **(8)** zum Verblocken der Knochenplatte **(1)** mittels des an der Knochenschraube **(9)** vorhandenen Verblockungsgewindes **(92)** vorhanden ist, **dadurch gekennzeichnet, dass**
f) die Eingriffskontur **(8)** besteht aus Konturtälern **(80)** und dazu benachbarten Konturspitzen **(81)**, die zumindest im wesentlichen horizontal angeordnet sind und partiell an der Wandung des Schraubenlochs **(4)** umlaufen, wobei es, auf die Höhe zur Plattenebene **(Y)** bezogen, zwischen einem Konturtal **(80)** und dem nächsten Konturtal **(80)** sowie zwischen einer Konturspitze **(81)** und der nächsten Konturspitze **(81)** keine Ausrichtung im Sinne eines Gewindes gibt.

2. Verblockbare Knochenplatte **(1,1')** nach Anspruch 1, **dadurch gekennzeichnet, dass** das Schraubenloch **(4)**
a) an der Plattenoberseite **(5)** eine kalottenförmige Ansenkung **(7)** zur partiellen Aufnahme der unteren Partie des Schraubenkopfes (90) und der Zentrierung der Knochenschraube **(9)** aufweist; und
b) an der Plattenunterseite **(6)** an seinem Austritt **(83)** langlochförmig ist.

3. Verblockbare Knochenplatte **(1,1')** nach Anspruch 1 oder 2, **dadurch gekennzeichnet, dass** das Verblockungsgewinde **(92)** der Knochenschraube **(9)**
a) ein- oder mehrgängig; und
b) von anderer oder gleicher Geometrie wie das Knochengewinde **(94)** ist; wobei
c) das Knochengewinde **(94)** selbstbohrend und/oder selbstschneidend sein kann.

4. Verblockbare Knochenplatte **(1,1')** nach einem der Ansprüche 1 bis 3, **dadurch gekennzeichnet, dass** die Konturtäler **(80)** und die Konturspitzen **(81)** in Richtung der Plattenlängsachse **(Z)** oder zu dieser im Winkel stehen.

5. Verblockbare Knochenplatte **(1,1')** nach einem der Ansprüche 1 bis 4, **dadurch gekennzeichnet, dass** die Eingriffskontur **(8)**
a) einem Spitz-, Rund-, Trapez- oder Sägengewinde ähnlich ist; und
b) vorzugsweise durch Fräsen erzeugt wird.

6. Verblockbare Knochenplatte **(1,1')** nach einem der Ansprüche 1 bis 5, **dadurch gekennzeichnet, dass** die Eingriffskontur **(8)**
a) an den horizontal gelegenen Enden ausläuft, wodurch dort im Schraubenloch **(4)** unkonturierte Wandungsbereiche **(82)** verbleiben; und
b) die unkonturierten Wandungsbereiche **(82)** vorzugsweise auf der theoretischen Hauptachse des im Bereich der Eingriffskontur **(8)** langlochförmigen Schraubenlochs **(4)** liegen.

7. Verblockbare Knochenplatte **(1,1')** nach einem der Ansprüche 1 bis 6, **dadurch gekennzeichnet, dass** in die Schraubenlöcher **(4)** die Knochenschrauben **(9)** vertikal oder mit einem Winkelversatz (α) in Relation zur Plattenlängsachse **(Z)** und Plattenebene **(Y)** einsetzbar sind.

8. Verblockbare Knochenplatte **(1,1')** nach einem der Ansprüche 1 bis 7, **dadurch gekennzeichnet, dass** bei der Verblockung am Verblockungsgewinde **(92)** und an der Eingriffskontur **(8)** durch Deformation Biegekanten (920,810) entstehen, die eine erhöhte Sicherung gegen selbständiges Lösen der Knochenschraube **(9)** bewirken.

9. Verblockbare Knochenplatte **(1,1')** nach einem der Ansprüche 1 bis 8, **dadurch gekennzeichnet, dass** der vertikale Abstand zwischen benachbarten Konturtälern **(80)** und Konturspitzen **(81)** vorzugsweise im Bereich zwischen 0.5 mm und 1.0 mm liegt.

10. Verblockbare Knochenplatte **(1,1')** nach einem der Ansprüche 1 bis 7, **dadurch gekennzeichnet, dass** in die Schraubenlöcher **(4)** auch Knochenschrauben **(19,29)** ohne Verblockungsgewinde **(92)** vertikal oder schräg einsetzbar sind.

11. Verblockbare Knochenplatte **(1')** nach einem der Ansprüche 1 bis 10, **dadurch gekennzeichnet, dass** die Plattenlängsachse **(Z)** der sich in der Plattenebene **(Y)** erstreckenden Knochenplatte **(1')** zumindest im wesentlichen kreisbogenförmig ist.

12. Verblockbare Knochenplatte **(1')** nach Anspruch 11, **dadurch gekennzeichnet, dass** sich durch die kreisbogenförmige Plattenlängsachse **(Z)** zwischen den Mittelachsen **(Z**_{**1**}**)** der Stege **(3)** und der Mittelachse **(Z**_{**2**}**)** des jeweils benachbarten Schraubenlochs **(4)** eine Versetzung (β) mit z.B. 1° ≤ β ≤ 10° und vorzugsweise β = 2.5° ergibt.

13. Verblockbare Knochenplatte **(1')** nach Anspruch 11 oder 12, **dadurch gekennzeichnet, dass** von der Vielzahl der vorhandenen Schraubenlöcher **(4)** nur einige die Eingriffskontur **(8)** zur Verblockung aufweisen, während die verbleibenden Schraubenlöcher **(4)** als zylindrische Standard-Schraubenlöcher und/oder als richtungsorientierte Kompressionslöcher ausgebildet sind.

14. Verblockbare Knochenplatte **(1')** nach einem der Ansprüche 11 bis 13, **dadurch gekennzeichnet, dass** die Knochenplatte **(1')**
a) aus einem Hauptsegment **(10')** mit kreisbogenförmiger Plattenlängsachse **(Z)** besteht; und
b) ein- oder beidseitig des Hauptsegments **(10')** ein sich ebenfalls in der Plattenebene **(Y)** erstreckendes Lateralsegment **(11')** ansetzt, welches gerade oder gebogen ist; wobei
c) der Übergang zwischen dem Hauptsegment (10') und dem Lateralsegment **(11')** von einem in der Plattenebene **(Y)** sich erstreckenden Winkelsegment **(12')** gebildet wird.

15. Verblockbare Knochenplatte **(1')** nach Anspruch 14, **dadurch gekennzeichnet, dass**
a) das Winkelsegment **(12')**
aa) lochfrei ist; oder
ab) zumindest ein Schraubenloch **(4)** hat, welches die Eingriffskontur **(8)** zur Verblockung aufweist oder ein zylindrisches Standard-Schraubenloch ist oder als richtungsorientiertes Kompressionsloch ausgebildet ist; und
ac) vorzugsweise gegenüber den die Plattenglieder **(2)** verbindenden Stegen **(3)** verstärkt ist; und
b) das Lateralsegment **(11')** mit Schraubenlöchern **(4)** versehen ist, welche die Eingriffskontur **(8)** zur Verblockung aufweisen oder zylindrische Standard-Schraubenlöcher sind oder als richtungsorientierte Kompressionslöcher ausgebildet sind.

16. Verblockbare Knochenplatte **(1')** nach einem der Ansprüche 11 bis 15, **dadurch gekennzeichnet, dass** die kreisbogenförmige Gestalt der Knochenplatte **(1')** ohne Umformen erzeugt wird.

17. Verblockbare Knochenplatte **(1')** nach einem der Ansprüche 11 bis 16, **dadurch gekennzeichnet, dass**
a) die Knochenplatte **(1')** zum im wesentlichen horizontalen, U-förmigen Umfassen des humanen Kieferknochens, insbesondere des Unterkiefers, konfiguriert ist; wobei
b) die Enden der Knochenplatte **(1'),** welche nur aus einem Hauptsegment **(10')** oder aus einem bzw. zwei zusätzlichen Lateralsegmenten **(11')** besteht, zum Übergreifen auf zumindest einen aufsteigenden Kieferast bemessen ist.

## Claims

1. A blockable bone plate (1, 1') with:
a) a plurality of plate members (2) which are connected to each other via waist-like webs (3) and lie on a plate plane (Y) in the unbent state;
b) a screw hole (4) in at least some plate members (2);
c) bone screws (9) which can be introduced through the screw holes (4) for securing the bone plate (1, 1'); where
d) the individual bone screw (9) has a screw head (90) which is thickened in diameter, a blocking thread (92) situated below this, and a bone thread (94) which extends at least partially over the screw shank (93), which has a smaller cross-sectional diameter than the screw head (90);
e) inside the individual screw hole (4), an engagement contour (8) is provided for blocking the bone plate (1) by means of the blocking thread (92) provided on the bone screw (9), **characterized in that**
f) the engagement contour (8) consists of contour valleys (80) and adjacent contour peaks (81) which are arranged at least substantially horizontally and partially run peripherally on the wall of the screw hole (4), and, in relation to the plate plane (Y), between one contour valley (80) and the next contour valley (80) and between one contour peak (81) and the next contour peak (81) there is no orientation in the sense of a thread.

2. The blockable bone plate (1, 1') as claimed in claim 1, **characterized in that** the screw hole (4)
a) has a spherical countersink (7) on the upper surface (5) of the plate for partially receiving the lower portion of the screw head (90) and for centering the bone screw (9); and
b) has the shape of an oblong hole at its exit (83) on the lower surface (6) of the plate.

3. The blockable bone plate (1, 1') as claimed in claim 1 or 2, **characterized in that** the blocking thread (92) of the bone screw (9)
a) is single-threaded or multi-threaded; and
b) can have a geometry different than or identical to the bone thread (94); where
c) the bone thread (94) can be self-boring and/or self-tapping.

4. The blockable bone plate (1, 1') as claimed in one of claims 1 through 3, **characterized in that** the contour valleys (80) and the contour peaks (81) are oriented in the direction of the longitudinal axis (Z) of the plate or at an angle to it.

5. The blockable bone plate (1, 1') as claimed in one of claims 1 through 4, **characterized in that** the engagement contour (8)
a) is similar to a pointed, round, trapezoidal or serrated thread; and
b) is preferably produced by milling.

6. The blockable bone plate (1, 1') as claimed in one of claims 1 through 5, **characterized in that** the engagement contour (8)
a) runs out at the horizontally located ends, by which means uncontoured wall areas (82) are left there in the screw hole (4); and
b) the uncontoured wall areas (82) preferably lie on the theoretical main axis of the oblong screw hole (4) in the area of the engagement contour (8).

7. The blockable bone plate (1, 1') as claimed in one of claims 1 through 6, **characterized in that** the bone screws (9) can be inserted into the screw holes (4) vertically or with an angular offset (α) in relation to the longitudinal axis (Z) of the plate and the plate plane (Y).

8. The blockable bone plate (1, 1') as claimed in one of claims 1 through 7, **characterized in that** bend edges (920, 810) develop by deformation on the blocking thread (92) and on the engagement contour (8) during blocking and these provide increased security against the bone screw (9) coming loose.

9. The blockable bone plate (1, 1') as claimed in one of claims 1 through 8, **characterized in that** the vertical distance between adjacent contour valleys (80) and contour peaks (81) lies preferably in the range between 0.5 mm and 1.0 mm.

10. The blockable bone plate (1, 1') as claimed in one of claims 1 through 7, **characterized in that** bone screws (19, 29) without blocking thread (92) can also be inserted vertically or obliquely into the screw holes (4).

11. The blockable bone plate (1') as claimed in one of claims 1 through 10, **characterized in that** the longitudinal axis (Z) of the bone plate (1') extending in the plate plane (Y) is at least substantially in the shape of an arc of a circle.

12. The blockable bone plate (1') as claimed in claim 11, **characterized in that**, as a result of the circular arc-shaped longitudinal axis (Z) of the plate, an offset (β) of for example 1° ≤ β ≤ 10° and preferably β = 2.5° is obtained between the center axes (Z₁) of the webs (3) and the center axis (Z₂) of the respectively adjacent screw hole (4).

13. The blockable bone plate (1') as claimed in claim 11 or 12, **characterized in that**, of the plurality of screw holes (4) present, only some have the engagement contour (8) for blocking, while the remaining screw holes (4) are designed as cylindrical standard screw holes and/or as oriented compression holes.

14. The blockable bone plate (1') as claimed in one of claims 11 through 13, **characterized in that** the bone plate (1')
a) consists of a main segment (10') with a plate longitudinal axis (Z) in the shape of an arc of a circle; and
b) adjoining the main segment (10') at one end or both ends there is a lateral segment (11') which likewise extends in the plate plane (Y) and which is straight or curved; where
c) the transition between the main segment (10') and the lateral segment (11') is formed by an angled segment (12') extending in the plate plane (Y).

15. The blockable bone plate (1') as claimed in claim 14, **characterized in that**
a) the angled segment (12')
aa) has no holes; or
ab) has at least one screw hole (4) which has the engagement contour (8) for blocking or is a cylindrical standard screw hole or is designed as an oriented compression hole; and
ac) is preferably strengthened relative to the webs (3) connecting the plate members (2); and
b) the lateral segment (11') is provided with screw holes (4) which have the engagement contour (8) for blocking or are cylindrical standard screw holes or are designed as oriented compression holes.

16. The blockable bone plate (1') as claimed in one of claims 11 through 15, **characterized in that** the circular arc shape of the bone plate (1') is produced without forming.

17. The blockable bone plate (1') as claimed in one of claims 11 through 16, **characterized in that**
a) the bone plate (1') is configured for the substantially horizontal, U-shaped engagement of the human jaw bone, in particular the lower jaw; where
b) the ends of the bone plate (1') which consists only of a main segment (10') or of one or two additional lateral segments (11') are dimensioned so as to engage over at least one ascending branch of the jaw.

## Revendications

1. Plaque d'ostéosynthèse (1, 1') pouvant être bloquée, comprenant :
a) plusieurs organes de plaque (2) qui sont connectés les uns aux autres par des nervures en forme de taille (3) et qui se trouvent dans l'état non cintré sur un plan de plaque (Y) ;
b) au moins à chaque fois un trou fileté (4) dans quelques organes de plaque (2) ;
c) des vis à os (9) pouvant être insérées à travers les trous filetés (4) pour la fixation de la plaque d'ostéosynthèse (1, 1') ;
d) la vis à os (9) individuelle présentant une tête de vis (90) de diamètre épaissi, un filetage de blocage (92) situé en dessous et un filetage d'os (94) qui s'étend au moins en partie sur la tige de la vis (93), qui possède un diamètre en section transversale inférieur à la tête de la vis (90) ;
e) un contour d'engagement (8) étant prévu à l'intérieur du trou fileté individuel (4) pour le blocage de la plaque d'ostéosynthèse (1) au moyen du filetage de blocage (92) prévu sur la vis à os (9), **caractérisée en ce que**
f) le contour d'engagement (8) se compose de vallées de contour (80) et de pointes de contour (81) adjacentes, qui sont disposées au moins essentiellement horizontalement et entourent partiellement la paroi du trou fileté (4), et aucune orientation au sens d'un filetage n'existe, par rapport à la hauteur au plan de la plaque (Y), entre une vallée de contour (80) et la vallée de contour (80) suivante ainsi qu'entre une pointe de contour (81) et la pointe de contour (81) suivante.

2. Plaque d'ostéosynthèse (1, 1') pouvant être bloquée selon la revendication 1, **caractérisée en ce que** le trou fileté (4)
a) présente sur le côté supérieur de la plaque (5) un chanfrein de forme sphérique (7) pour la réception partielle de la partie inférieure de la tête de vis (90) et pour le centrage de la vis à os (9) ; et
b) est en forme de trou oblong au niveau de sa sortie (83) sur le côté inférieur de la plaque (6).

3. Plaque d'ostéosynthèse (1, 1') pouvant être bloquée selon la revendication 1 ou 2, **caractérisée en ce que** le filetage de blocage (92) de la vis à os (9)
a) est à pas simple ou à pas multiple ; et
b) a une géométrie différente ou identique à celle du filetage d'os (94) ;
c) le filetage d'os (94) pouvant être auto-perforant et/ou auto-taraudeur.

4. Plaque d'ostéosynthèse (1, 1') pouvant être bloquée selon l'une quelconque des revendications 1 à 3, **caractérisée en ce que** les vallées de contour (80) et les pointes de contour (81) se situent dans la direction de l'axe longitudinal (Z) de la plaque ou suivant un angle par rapport à celui-ci.

5. Plaque d'ostéosynthèse (1, 1') pouvant être bloquée selon l'une quelconque des revendications 1 à 4, **caractérisée en ce que** le contour d'engagement (8)
a) est similaire à un filetage en pointe, arrondi, de forme trapézoïdale ou en dents de scie ; et
b) est produit de préférence par fraisage.

6. Plaque d'ostéosynthèse (1, 1') pouvant être bloquée selon l'une quelconque des revendications 1 à 5, **caractérisée en ce que** le contour d'engagement (8)
a) se termine au niveau des extrémités situées horizontalement, ce qui fait qu'il subsiste à cet endroit des zones de paroi (82) non contourées dans le trou fileté (4) ; et
b) les zones de paroi (82) non contourées se situent de préférence sur l'axe principal théorique du trou fileté (4) en forme de trou oblong dans la région du contour d'engagement (8).

7. Plaque d'ostéosynthèse (1, 1') pouvant être bloquée selon l'une quelconque des revendications 1 à 6, **caractérisée en ce que** les vis à os (9) peuvent être insérées dans les trous filetés (4) verticalement ou avec un déport angulaire (α) par rapport à l'axe longitudinal (Z) de la plaque et au plan de la plaque (Y).

8. Plaque d'ostéosynthèse (1, 1') pouvant être bloquée selon l'une quelconque des revendications 1 à 7, **caractérisée en ce que** lors du blocage sur le filetage de blocage (92) et sur le contour d'engagement (8), il se produit par déformation des arêtes de flexion (920, 810) qui provoquent une fixation améliorée contre un desserrage autonome de la vis à os (9).

9. Plaque d'ostéosynthèse (1, 1') pouvant être bloquée selon l'une quelconque des revendications 1 à 8, **caractérisée en ce que** la distance verticale entre des vallées de contour (80) et des pointes de contour (81) adjacentes est de préférence comprise entre 0,5 mm et 1,0 mm.

10. Plaque d'ostéosynthèse (1, 1') pouvant être bloquée selon l'une quelconque des revendications 1 à 7, **caractérisée en ce que** l'on peut insérer verticalement ou en biais dans les trous filetés (4) également des vis à os (19, 29) sans filetage de blocage (92).

11. Plaque d'ostéosynthèse (1') pouvant être bloquée selon l'une quelconque des revendications 1 à 10, **caractérisée en ce que** l'axe longitudinal (Z) de la plaque d'ostéosynthèse (1') s'étendant dans le plan de la plaque (Y) est au moins essentiellement en forme d'arc de cercle.

12. Plaque d'ostéosynthèse (1') pouvant être bloquée selon la revendication 11, **caractérisée en ce qu'** un déport (β) avec par exemple 1° ≤ β ≤ 10°, et de préférence β = 2,5°, est obtenu par l'axe longitudinal en forme d'arc de cercle (Z) de la plaque entre les axes médians (Z₁) des nervures (3) et l'axe médian (Z₂) de chaque trou fileté respectif adjacent (4).

13. Plaque d'ostéosynthèse (1') pouvant être bloquée selon la revendication 11 ou 12, **caractérisée en ce que** parmi la pluralité de trous filetés (4) prévus, seulement quelques-uns présentent le contour d'engagement (8) pour le blocage, tandis que les trous filetés (4) restants sont réalisés sous forme de trous filetés cylindriques standard et/ou sous forme de trous de compression orientés.

14. Plaque d'ostéosynthèse (1') pouvant être bloquée selon l'une quelconque des revendications 11 à 13, **caractérisée en ce que** la plaque d'ostéosynthèse (1')
a) se compose d'un segment principal (10') avec un axe longitudinal (Z) de plaque en forme d'arc de cercle ; et
b) possède, attaché à elle, un segment latéral (11') s'étendant également dans le plan de la plaque (Y), d'un côté ou des deux côtés du segment principal (10'), lequel est droit ou cintré ;
c) la transition entre le segment principal (10') et le segment latéral (11') étant formée par un segment angulaire (12') s'étendant dans le plan de la plaque (Y).

15. Plaque d'ostéosynthèse (1') pouvant être bloquée selon la revendication 14, **caractérisée en ce que**
a) le segment angulaire (12')
aa) est exempt de trou ; ou
ab) a au moins un trou fileté (4) qui présente le contour d'engagement (8) pour le blocage ou est un trou fileté cylindrique standard ou est réalisé sous forme de trou de compression orienté ; et
ac) est renforcé de préférence par rapport aux nervures (3) reliant les organes de plaque (2) ; et
b) le segment latéral (11') est pourvu de trous filetés (4) qui présentent le contour d'engagement (8) pour le blocage ou qui sont des trous filetés cylindriques standard ou qui sont réalisés sous forme de trous de compression orientés.

16. Plaque d'ostéosynthèse (1') pouvant être bloquée selon l'une quelconque des revendications 11 à 15, **caractérisée en ce que** la forme en arc de cercle de la plaque d'ostéosynthèse (1') est produite sans formage.

17. Plaque d'ostéosynthèse (1') pouvant être bloquée selon l'une quelconque des revendications 11 à 16, **caractérisée en ce que**
a) la plaque d'ostéosynthèse (1') est configurée pour comprendre essentiellement horizontalement, en forme de U, l'os de la mâchoire humaine, notamment de la mâchoire inférieure ;
b) les extrémités de la plaque d'ostéosynthèse (1'), qui ne se compose que d'un segment principal (10') ou d'un ou de deux segments latéraux supplémentaires (11'), sont dimensionnées pour venir en prise par le dessus avec au moins une branche de mâchoire montante.
